## Europäisches Patentamt
## European Patent Office
## Office européen des brevets

(19)

(11) Numéro de publication: **0 241 361**
**B1**

(12) **FASCICULE DE BREVET EUROPEEN**

(45) Date de publication du fascicule du brevet:
29.11.89

(51) Int. Cl.⁴: **A61F 2/34**

(21) Numéro de dépôt: 87400737.0

(22) Date de dépôt: 03.04.87

(54) **Prothèse de hanche.**

(30) Priorité: **04.04.86 FR 8604874**

(43) Date de publication de la demande:
**14.10.87 Bulletin 87/42**

(45) Mention de la délivrance du brevet:
**29.11.89 Bulletin 89/48**

(84) Etats contractants désignés:
**AT BE CH DE ES GB GR IT LI LU NL SE**

(56) Documents cités:
**FR-A- 1 552 585**
**US-A- 3 840 904**

(73) Titulaire: **Demeulenaere, Claude, Montplaisir 7 - 28, rue Wauthier, F-78100 St-Germain-en-Laye(FR)**

(72) Inventeur: **Demeulenaere, Claude, Montplaisir 7 - 28, rue Wauthier, F-78100 St-Germain-en-Laye(FR)**

(74) Mandataire: **Bruder, Michel, 10 rue de la Pépinière, F-75008 Paris(FR)**

## Description

La présente invention concerne une prothèse de hanche.

On utilise actuellement en chirurgie des prothèses de hanche qui sont constituées de trois pièces à savoir une cupule cotyloïdienne, une queue fémorale métallique comprenant un col et une tête fémorale sphérique solidaire ou indépendante du col.Ces prothèses peuvent être fixées à l'os soit par scellement grâce à du ciment plastique soit directement sans ciment par divers moyens plus ou moins valables.

Elles nécessitent toutes un creusement plus ou moins important de l'os qui fragilise ce dernier, en particulier au niveau du cotyle. De plus, le scellement au ciment peut entraîner des descellements au bout d'un temps variable.

La présente invention vise à améliorer les résultats des prothèses de hanche en proposant une prothèse cotyloïdienne non cimentée dont l'ancrage ne nécessite aucun creusement de l'os

A cet effet cette prothèse de hanche comprenant une cupule cotyloïdienne externe métallique, de forme sensiblement hémishpérique, fixée dans l'os iliaque, un noyau cotyloïdien interne en matière plastique,emboité dans la cupule cotyloïdienne externe métallique, une queue fémorale emboîtée dans la diaphyse fémorale, une tête fémorale sphérique engagée dans le noyau cotyloïdien interne et un col de liaison entre la tête et la queue fémorale, est caractérisée en ce que la cupule cotyloïdienne métallique externe est solidaire d'un aileron métallique plan s'encastrant à frottement dur dans une fente de l'os iliaque, le plan de cet aileron formant, avec le plan d'ouverture du cotyle, un angle allant de 50 à 80° et de préférence d'environ 65°.

Le noyau cotyloïdien interne en polyéthylène peut être symétrique ou encore présente un plan asymétrique avec un plan d'ouverture du cotyle faisant un angle variant de 90 à 120° avec l'axe du noyau cotyloïdien.Cette caractéristique permet de corriger un éventuel défaut d'antéversion ou de rétroversion du cotyle.

Le col fémoral est de préférence amovible et il peut être coudé d'un angle variant de 10° à 30°. Cette disposition permet d'ajuster et éventuellement de corriger une antéversion ou d'une rétroversion de la queue fémorale.

On décrira ci-après,à titre d'exemples non limitatifs, diverses formes d'exécution de la présente invention, en référence au dessin annexé sur lequel :

La figure 1 est une vue de profil d'une prothèse de hanche en place , dans le plan de l'aileron de la cupule cotyloïdienne externe.

La figure 2 est une vue de face de la prothèse de hanche en place.

La figure 3 est une vue de l'os iliaque, montrant le plan de coupe de la fente destinée à recevoir l'aileron.

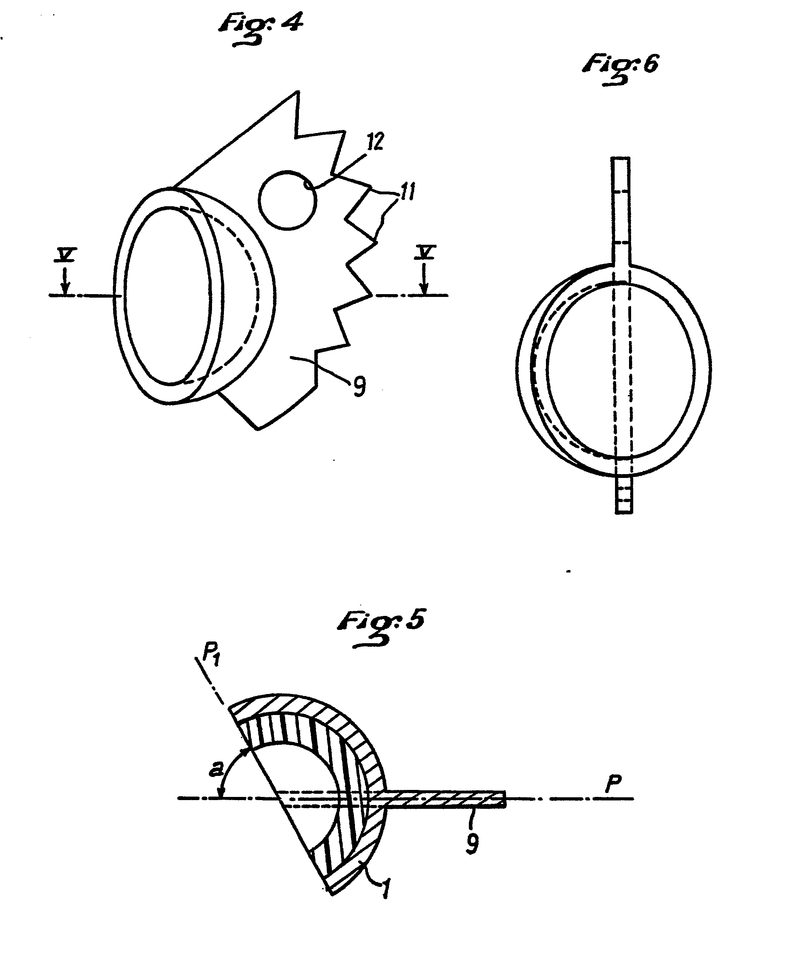

La figure 4 est une vue en élévation de la cupule cotyloïdienne externe métallique de la prothèse de hanche suivant l'invention, perpendiculairement au plan de l'aileron externe.

La figure 5 est une vue en coupe faite suivant la ligne V-V de la figure 4, le noyau cotyloïdien interne en polyéthylène étant représenté en place dans la cupule métallique externe.

La figure 6 est une vue de la cupule cotyloïdienne externe , prise de la gauche sur la figure 4 et regardant l'aileron par la tranche.

Les figures 7 et 8 sont des vues en coupe de divers noyaux cotyloïdiens en polyéthylène.

Les figures 9 et 10 sont des vues schématiques illustrant des cols fémoraux amovibles.

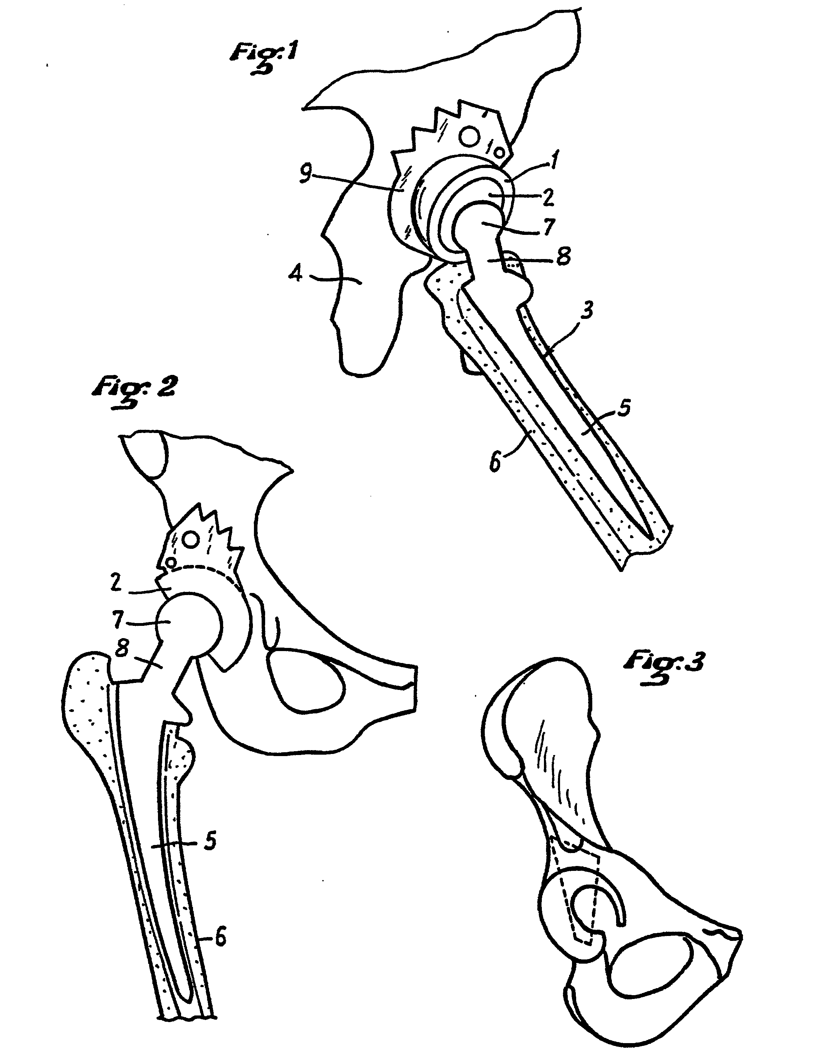

La prothèse de hanche suivant l'invention qui est représentée sur les figures 1 et 2, comprend essentiellement cinq pièces, à savoir une cupule cotyloïdienne externe métallique 1 fixée dans l'os iliaque 4, un noyau cotyloïdien interne en matière plastique 2, emboité dans la cupule métallique externe 1, une queue fémorale 5 emboîtée dans la diaphyse fémorale 6, une tête fémorale sphérique 7 logée dans le noyau cotyloïdien interne en matière plastique 2, et un col fémoral 8 assurant la liaison entre la tête 7 et la queue fémorale 5.

Suivant l'invention la cupule cotyloïdienne métallique externe 1 est encastrée dans l'aile iliaque et dans l'ischion au moyen d'un aileron 9 qui s'étend vers la grande échancrure sciatique et qui est contenu dans un plan diamétral P (figure 5) de la cupule cotyloïdienne externe 1 hémisphérique. Ce plan P forme, avec le plan frontal $P_1$ contenant l'ouverture du cotyle , un angle $\underline{a}$ qui est compris entre 50 et 80° et qui est de préférence égal à 65°. Cet aileron 9, de faible épaisseur, est fixé par emboîtement dans une fente de l'os de largeur sensiblement égale à l'épaisseur de l'aileron 9, fente dans laquelle il tient par simple frottement.

Pour améliorer la fixation de cet aileron d'ancrage 9 ,on peut prévoir, sur son bord périphérique 9, une découpe en dents de scie 11. Par ailleurs l'aileron 9 peut être percé d'un ou plusieurs trous 12 à travers lesquels peut se reconstituer l'os, en renforcant ainsi l'ancrage de la cupule cotyloïdienne 1.

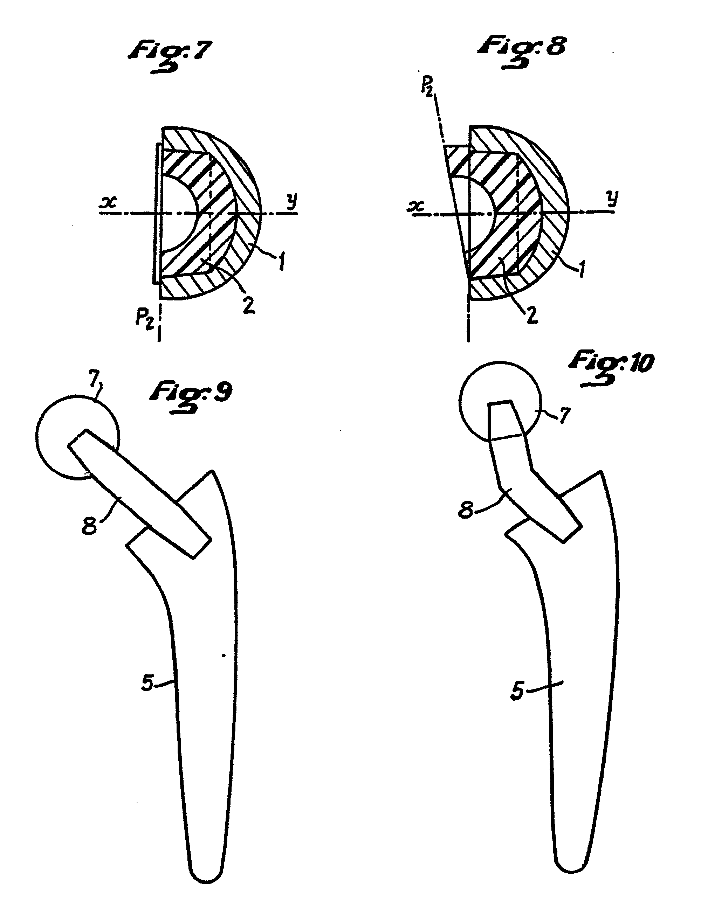

Suivant une caractéristique complémentaire de l'invention le noyau cotyloïdien interne 2 en polyéthylène présente un plan frontal d'antéversion, c'est-à-dire celui contenant l'ouverture du cotyle, qui fait un angle pouvant varier de 90° à 120° avec l'axe xy du noyau cotyloïdien comme il est représenté sur les figures 7 et 8. Cette disposition permet de mieux adapter la prothèse de hanche suivant l'invention aux conditions d'implantation particulières rencontrées.

La prothèse de hanche suivant l'invention peut également comporter avantageusement, ainsi qu'il est représenté sur les figures 9 et 10, un col fémoral 8 amovible indépendant de la queue fémorale 5 et donc interchangeable. Ce col fémoral interchangeable 8 peut être rectiligne, de longueur variable ainsi qu'il est représenté sur la figure 9, ou encore il peut être coudé, avec un angle allant de 10 à 30°, afin de permettre une modification de l'angle d'antéversion du col si nécessaire.

## Revendications

1.- Prothèse de hanche comprenant une cupule cotyloïdienne externe métallique (1), de forme sensiblement hémisphérique fixée dans l'os iliaque , un noyau cotyloïdien interne (2) en matière plastique, emboîté dans la cupule cotyloïdienne externe métallique (1), une queue fémorale (5) emboîtée dans la diaphyse fémorale (6) , une tête fémorale sphérique (7) engagée dans le noyau cotyloïdien interne (2) et un col de liaison (8) entre la tête et la queue fémorale, caractérisée en ce que la cupule cotyloïdienne métallique externe (1) est solidaire d'un aileron métallique (6) s'encastrant à frottement dur dans une fente de l'os iliaque , le plan (P) de cet aileron (6) formant, avec le plan d'ouverture (P₁) du cotyle, un angle (a) allant de 50 à 80° et de préférence d'environ 65°.

2.- Prothèse de hanche suivant la revendication 1 caractérisée en ce que l'aileron (9) présente, sur son bord périphérique, une découpe en dents de scie (11).

3.- Prothèse de hanche suivant l'une quelconque des revendications précédentes caractérisée en ce que l'aileron (9) est percé d'au moins un trou (12).

4.- Prothèse de hanche suivant l'une quelconque des revendications précédentes caractérisée en ce qu'elle comprend un noyau cotyloïdien interne en polyétylène (2) dont le plan (P₂) contenant l'ouverture du cotyle forme un angle allant de 90 à 120° avec l'axe (xy)du noyau cotyloïdien.

5.- Prothèse de hanche suivant l'une quelconque des revendications précédentes caractérisée en ce qu'elle comporte un col fémoral (8) amovible et interchangeable.

6.- Prothèse de hanche suivant la revendication 5 caractérisée en ce que le col fémoral interchangeable (8) est rectiligne.

7.- Prothèse de hanche suivant la revendication 5 caractérisée en ce que le col fémoral interchangeable (8) est coudé suivant un angle de 10 à 30°.

## Claims

1. Hip prosthesis comprising a metallic external cotyloid cup (1) of essentially hemispherical shape fixed in the iliac bone, an internal cotyloid core (2) of plastic material fitted in the metallic external cotyloid cup (1), a femoral tail (5) fitted in the femoral diaphysis (6), a spherical femoral head (7) engaged in the internal cotyloid core (2) and a joining neck (8) between the femoral head and the femoral tail, characterized in that the external metallic cotyloid cup (1) is integral with a metallic flap (9) fitting into a slot in the iliac bone with tight friction, the plane (P) of this flap (9) forming, with the plane of opening (P₁) of the cotyloid, an angle (a) ranging from 50 to 80° and preferably of about 65°.

2. Hip prosthesis according to Claim 1, characterized in that the flap (9) has, on its peripheral edge, a saw-tooth cutting (11).

3. Hip prosthesis according to either one of the preceding claims, characterized in that the flap (9) is pierced with at least one hole (12).

4. Hip prosthesis according to any one of the preceding claims, characterized in that it comprises an internal cotyloid core (2) of polyethylene, of which the plane (P₂) containing the opening of the cotyloid forms an angle ranging from 90 to 120° with the axis (xy) of the cotyloid core.

5. Hip prosthesis according to any one of the preceding claims, characterized in that it comprises a removable and interchangeable femoral neck (8).

6. Hip prosthesis according to Claim 5, characterized in that the interchangeable femoral neck (8) is rectilinear.

7. Hip prosthesis according to Claim 5, characterized in that the interchangeable femoral neck (8) is bent at an angle of 10 to 30°.

## Patentansprüche

1. Hüftprothese mit einer der Gelenkpfanne zugeordneten, äußeren, metallischen Schale von im wesentlichen halbkugeliger Gestalt, die am Os Ilium befestigt ist, mit einem der Gelenkpfanne zugeordneten inneren Kern (2) aus Kunststoffmaterial, der in diese der Gelenkpfanne zugeordnete, äußere, metallische Schale eingefügt ist, mit einem femoralen Stiel (5), der in die femorale Diaphyse (6) eingebettet ist, mit einem sphärischen, femoralen Kopf (7), der in den inneren, der Gelenkpfanne zugeordneten Kern (2) eingreift, und mit einem Verbindungshals (8) zwischen dem Kopf und dem femoralen Stiel, dadurch gekennzeichnet, daß die der Gelenkpfanne zugeordnete, äußere, metallische Schale (1) fest mit einem metallischen Flügel (9) verbunden ist, der mit harter Reibung in eine Ausnehmung im Os Ilium eingelassen ist, wobei die Ebene (P) dieses Flügels (9) mit der Öffnungsebene (P₁) der Gelenkpfanne einen Winkel (a) zwischen 50 und 80°, und vorzugsweise in der Größenordnung von 65° einschließt.

2. Hüftprothese nach Anspruch 1, dadurch gekennzeichnet, daß der Flügel (9) an seinem Umfangsrand einen sägezahnartigen Zuschnitt (11) aufweist.

3. Hüftprothese nach einem der vorhergehenden Ansprüche, dadurch gekennzeichnet, daß der Flügel (9) von zumindest einer Öffnung (12) durchbrochen wird.

4. Hüftprothese nach einem der vorhergehenden Ansprüche, dadurch gekennzeichnet, daß sie einen der Gelenkpfanne zugeordneten inneren Kern (2) aus Polyethylen umfaßt, dessen die Öffnung der Gelenkpfanne enthaltende Ebene (P₂) einen Winkel von 90 bis 120° mit der Achse (xy) des der Gelenkpfanne zugeordneten Kerns bildet.

5. Hüftprothese nach einem der vorhergehenden Ansprüche, dadurch gekennzeichnet, daß sie einen abnehmbaren und austauschbaren femoralen Hals (8) umfaßt.

6. Hüftprothese nach Anspruch 5, dadurch gekennzeichnet, daß der austauschbare femorale Hals (8) geradlinig ist.

7. Hüftprothese nach Anspruch 5, dadurch gekennzeichnet, daß der austauschbare femorale Hals (8) mit einem Winkel zwischen 10 und 30° knieförmig abgebogen ist.

EP 0 241 361 B1

Fig:1

9

1

2

7

8

4

3

5

6

Fig: 2

2

7

8

5

6

Fig:3

*Fig: 4*

*Fig: 6*

*Fig: 5*

*Fig. 7*

*Fig. 8*

*Fig. 9*

*Fig. 10*